# EUROPÄISCHE PATENTSCHRIFT

(11) **EP 1 457 161 B1**
(45) Veröffentlichungstag und Bekanntmachung des Hinweises auf die Patenterteilung: **17.01.2007**
(21) Anmeldenummer: 04003821.8
(22) Anmeldetag: 19.02.2004
(51) Int. Cl.: A61B 17/70

(54) **Verankerungselement zur Verwendung in der Wirbelsäulen- oder Knochenchirurgie und Verfahren zu seiner Herstellung**
Fixation device for use in spinal or bone surgery and method of its manufacture
Dispositif de fixation pour l'utilisation dans la chirurgie de la colonne vertebrale ou de l'os et méthode de la fabrication

(30) Priorität: 11.03.2003 DE 10310540
(43) Veröffentlichungstag der Anmeldung: 15.09.2004
(73) Patentinhaber: BIEDERMANN MOTECH GmbH, 78054 VS-Schwenningen (DE)
(72) Erfinder: Biedermann, Lutz, 78048 VS-Villingen (DE); Harms, Jürgen, 76227 Karlsruhe (DE)
(74) Vertreter: Hofer, Dorothea

(56) Entgegenhaltungen:
- DE-A- 10 136 129
- DE-C- 10 157 969
- DE-U- 29 810 798

## Beschreibung

Die Erfindung betrifft ein Verankerungselement zur Verwendung in der Wirbelsäulen- oder Knochenchirurgie nach dem Oberbegriff des Patentanspruches 1 und ein Verfahren zu seiner Herstellung.

Ein Verankerungselement nach dem Oberbegriff des Patentanspruches 1 ist z.B. aus der US 5,005,562 in Form einer Monoaxial-Knochenschraube und aus der DE 43 07 576 C1 in Form einer Polyaxial-Knochenschraube bekannt. Die bekannten Verankerungselemente weisen ein Aufnahmeteil zur Aufnahme eines mehrere derartige Verankerungselemente verbindenden Stabes, ein mit dem Aufnahmeteil monoaxial bzw. polyaxial verbundenes Schraubenelement und eine zwischen die Schenkel des Aufnahmeteils einzuschraubende Innenschraube zur Fixierung des Stabes auf. Das Aufnahmeteil gemäß der DE 43 07 576 C1 besitzt ferner eine auf das Aufnahmeteil aufschraubbare Außenmutter zur zusätzlichen Fixierung.

Bei den bekannten Verankerungselementen weisen das Innengewinde bzw. das Außengewinde des Aufnahmeteils einen Gewindeauslauf auf. Der Abstand zwischen der Stabauflage innerhalb des Aufnahmeteils und dem Gewindeauslauf ist kleiner als der Durchmesser des aufzunehmenden Stabes. Dadurch wird gewährleistet, daß die Innenschraube oder die Außenmutter eine ausreichende Klemmkraft auf den Stab ausüben kann.

Aus der DE 298 10 798 U1 ist eine Knochenschraube bekannt, die einen Gabelkopf aufweist, der das Einsetzen eines Stützstabes ermöglicht. Es sind zwei verschiedene Gewinde für eine Innenschraube jeweils in einer teilweisen Schnittdarstellung gezeigt. Die unteren Ausläufe der Gewinde sind der Offenbarung nicht zu entnehmen.

Für einen vorgegebenen Stabdurchmesser ist die Länge der freien Schenkel des Aufnahmeteils und damit die Bauhöhe des Verankerungselements von der Stabauflage aus durch die für eine zuverlässige Fixierung erforderliche Strecke auf der das Innen- bzw. Außengewindes ausgebildet ist, bestimmt. Im Bereich des Gewindeauslaufs, der mehr als einen Gewindezahn enthalten kann, nimmt die Gewindetiefe ab und das Profil der Gewindezähne ist unscharf. Um eine ausreichende Festigkeit der Innenschraube bzw. der Außenmutter zu erhalten, muß sichergestellt werden, daß eine ausreichende Zahl von Gewindegängen mit tiefem Profil vorhanden ist. Somit ist entweder der Gewindeauslauf entsprechend tief in Richtung der Stabauflage herunterzuverlegen, oder nach oben in Richtung des freien Endes der Schenkel sind entsprechend viele Gewindegänge mit tiefem Profil vorzusehen, was die Bauhöhe erhöht.

Ferner ist die Fertigung des Gewindes mit Gewindeauslauf herstellungstechnisch aufwendig.

Es ist Aufgabe der Erfindung, ein verbessertes Verankerungselement der eingangs beschriebenen Art bereitzustellen, das einfach zu fertigen ist und eine verringerte Bauhöhe hat, jedoch die gleiche Sicherheit gegen Lockerung der Stabfixierung bietet, wie die herkömmlichen Verankerungselemente. Außerdem soll ein Verfahren zu dessen Herstellung bereitgestellt werden.

Die Aufgabe wird durch ein Verankerungselement gemäß Patentanspruch 1 bzw. durch ein Verfahren nach Anspruch 8 gelöst. Weiterbildungen der Erfindung sind in den Unteransprüchen angegegeben.

Das erfindungsgemäße Verankerungselement weist den Vorteil auf, daß es kostengünstiger herzustellen ist, da das Gewindeschneiden herstellungstechnisch gesehen weniger Aufwand erfordert. Ferner ist das Aufnahmeteil um die bei den herkömmlichen Veran-kerungselementen vorhandenen schwachen Auslaufgewindezähne in seiner Höhe reduziert.

Weitere Merkmale und Zweckmäßigkeiten der Erfindung ergeben sich aus der Beschreibung von Ausführungsbeispielen anhand der Figuren.

Von den Figuren zeigen:
- Fig. 1: eine Schnittdarstellung einer ersten Ausführungsform;
- Fig. 2: eine Schnittdarstellung einer zweiten Ausführungsform; und
- Fig. 3: eine Schnittdarstellung einer dritten Ausführungsform.

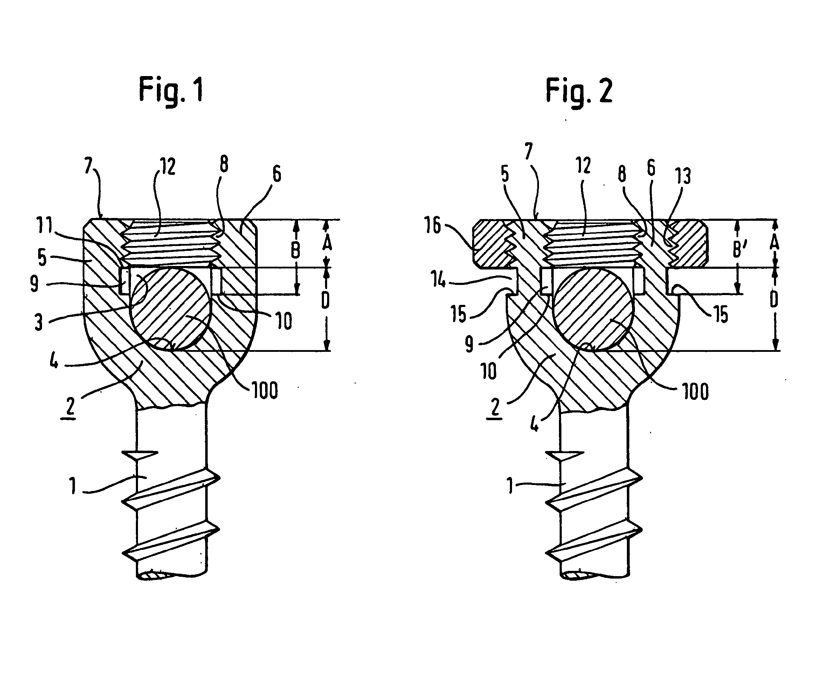

Fig. 1 zeigt eine Monoaxial-Knochenschraube als eine erste Ausführungsform eines Knochenverankerungselements. Diese weist einen Schaft 1 mit einem Knochengewindeabschnitt und ein mit dem Schaft starr verbundenes im wesentlichen zylindrisches Aufnahmeteil 2 zur Aufnahme eines die Knochenschraube mit weiteren Knochenschrauben zu verbindenden Stabes 100 eines vorgegebenen Durchmessers D auf. Hierzu besitzt das Aufnahmeteil 2 eine Ausnehmung 3 mit einem U-förmigem Querschnitt, die gerade so groß bemessen ist, daß der Stab 100 einlegbar ist und in dem durch den Grund 4 der Ausnehmung und die Seitenwände gebildeten Kanal gehalten ist. Durch die U-förmige Ausnehmung 3 sind zwei Schenkel 5, 6 gebildet, deren freies Ende 7 den oberen Rand des Aufnahmeteils bildet. Im eingelegten Zustand, in dem der Stab am Grund 4 der U-förmigen Ausnehmung aufliegt, besitzt die Staboberfläche einen vorbestimmten Abstand A von dem freien Ende 7 des Aufnahmeteils.

Ausgehend von dem freien Ende 7 erstreckt sich an den Schenkeln 5, 6 ein Innengewinde 8 über eine Strecke, die in dem gezeigten Ausführungsbeispiel gleich dem vorbestimmten Abstand A ist. Das Innengewinde ist hier als metrisches Gewinde ausgebildet. Angrenzend an das Innengewinde schließt sich eine in Umfangsrichtung verlaufende Ausnehmung bzw. ein Freistich 9 an, dessen dem Gewinde 8 abgewandter unterer Rand 10 von dem freien Ende 7 einen Abstand B aufweist, der größer ist, als der vorbestimmte Abstand A. Somit befindet sich der untere Rand 10 des Freistichs in einem Abstand vom Grund 4 der Ausnehmung, der kleiner ist, als der Stabdurchmesser D. In dem gezeigten Ausführungsbeispiel befindet sich der dem freien Ende 7 zugewandte obere Rand 11 des Freistichs auf der Höhe der Staboberfläche und weist somit den Abstand A vom freien Ende auf. Die Tiefe des Freistichs 9 ist bevorzugt gleich der Tiefe des Gewindes 8. Der Querschnitt des Freistichs 9 ist im wesentlichen rechteckig ausgebildet.

Ferner ist ein Sicherungselement in Form einer Innenschraube 12 zum Fixieren des Stabs in dem Aufnahmeteil vorgesehen, die ein mit dem Innengewinde zusammenwirkendes Außengewinde aufweist.

Im Betrieb wird zuerst die Knochenschraube in den Knochen eingeschraubt und anschließend der Stab eingelegt. Dann wird die Innenschraube 11 eingeschraubt bis ihre dem Stab zugewandte Unterseite auf den Stab drückt und diesen in seiner Position fixiert.

Die Herstellung der Knochenschraube erfolgt bevorzugt so, daß bei dem Aufnahmeteil in einem ersten Arbeitsgang der Freistich 9 erzeugt wird und in einem zweiten Arbeitsgang das Gewinde 8 aufgebracht wird. Im Vergleich zu den herkömmlichen Verankerungselementen mit Gewindeauslauf entstehen bei der Herstellung weniger Gewindegänge. Diese haben jedoch ein klares Profil und somit hohe Tragkraft. Somit kann das gesamte Gewinde 8 um die Strecke der bei den herkömmlichen Verankerungselementen mit Gewindeauslauf vorhandenen schwachen Auslaufgewindezähne in Richtung des Grunds 4 nach unten versetzt werden. Daher ist die Bauhöhe des Aufnahmeteils im Vergleich zu den herkömmlichen Verankerungselementen verringert. Die Herstellung ist ferner kostengünstiger, da durch das Fehlen des Gewindeauslaufs weniger Arbeitsaufwand beim Gewindeschneiden anfällt.

In einer Abwandlung erstreckt sich das Innengewinde 8 von dem freien Ende 7 aus über eine Strecke die kleiner ist, als der vorbestimmte Abstand A, so daß der obere Rand 11 des Freistichs 9 bei eingelegtem Stab 100 in Richtung auf das freie Ende 7 gesehen oberhalb der Staboberfläche liegt. In einer weiteren Abwandlung hat der Freistich 9 abgerundete Ecken oder sein oberer und unterer Rand sind angeschrägt.

In einer weiteren Abwandlung ist das Innengewinde anstelle als metrisches Gewinde als Sägengewinde, insbesondere als Sägengewinde mit einer horizontale Lastflanke, als Flachgewinde mit zwei horizontalen Flanken oder als Gewinde mit einem negativen Lastflankenwinkel ausgebildet. Die Innenschraube 12 weist dann ein dazu passendes Außengewinde auf. Die Gewinde können als Rechts- oder als Linksgewinde ausgebildet sein.

Die in Fig. 2 gezeigte zweite Ausführungsform unterscheidet sich von der in Fig. 1 gezeigten Ausführungsform dadurch, daß zusätzlich an den freien Schenkeln 5, 6 ein Außengewinde 13 vorgesehen ist, das sich vom freien Ende 7 aus gesehen bis zu einem vorbestimmten Abstand aus erstreckt, der in dem gezeigten Ausführungsbeispiel gleich dem Abstand A der Staboberfläche ist. Angrenzend an das Außengewinde ist ein Freistich 14 vorgesehen, dessen unterer, dem Grund 4 zugewandter Rand 15 von dem freien Ende 7 einen Abstand B' aufweist, der größer ist, als der vorbestimmte Abstand A. Die Tiefe des Freistichs ist bevorzugt gleich der Tiefe des Außengewindes. Es ist ferner als weiteres Sicherungselement eine Außenmutter 16 vorgesehen, die ein mit dem Außengewinde 13 zusammenwirkendes Innengewinde aufweist.

Der Betrieb erfolgt wie bei der ersten Ausführungsform, jedoch mit dem zusätzlichen Schritt des Aufschraubens des Außenmutter als zusätzliches Sicherungselement.

Die Herstellung des Verankerungselements umfaßt die zur ersten Ausführungsform zusätzlichen Schritte des Anbringens des Freistichs 14 und des Außengewindes 13, wobei wiederum der Freistich zuerst erzeugt wird und das Außengewinde anschließend.

In einer Abwandlung der zweiten Ausführungsform unterscheidet sich die Länge der Strecke auf der das Innengewinde 8 ausgebildet ist von der auf der das Außengewinde 13 ausgebildet ist. Entscheidend ist, daß das Innengewinde bzw. das Außengewinde sich vom freien Ende 7 aus nur bis höchstens zur Höhe Staboberfläche erstrecken und daß der untere Rand des Freistichs 9 bzw. 13 stets unterhalb der Staboberfläche liegt. In einer Abwandlung ist die Höhe des Freistichs unterschiedlich für das Innengewinde bzw. das Außengewinde.

In einer weiteren Abwandlung fehlt das Innengewinde und der daran angrenzende Freistich sowie die Innenschraube als Sicherungselement und es ist nur das Außengewinde mit dem daran angrenzenden Freistich vorgesehen. In einer weiteren Abwandlung ist ein Freistich nur angrenzend an eines der beiden Gewinde, Innengewinde oder Außengewinde vorgesehen.

Alle im Zusammenhang mit der ersten Ausführungsform beschriebenen Abwandlungen sind ferner auch bei der zweiten Ausführungsform anwendbar.

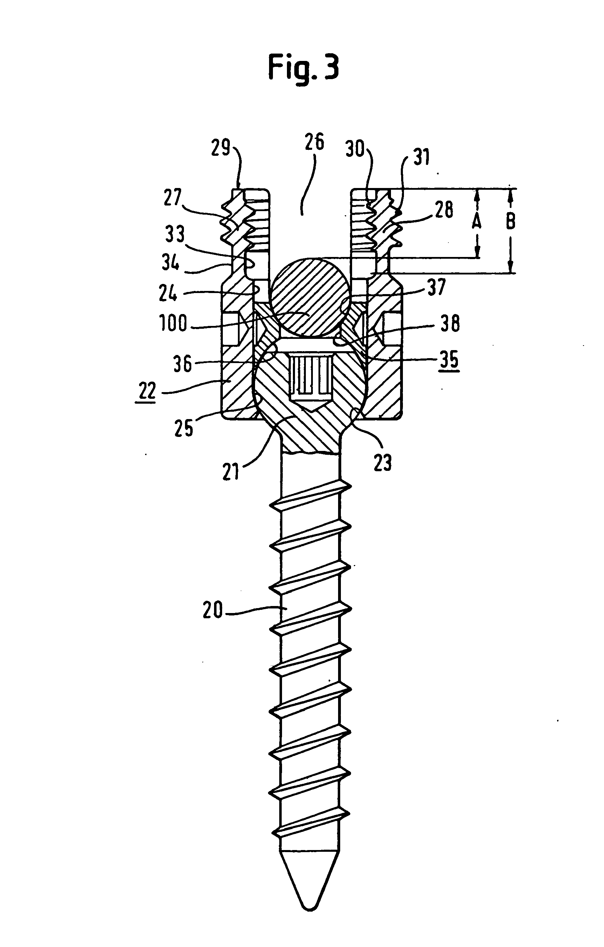

Fig. 3 zeigt eine dritte Ausführungsform, bei der das Verankerungselement als Polyaxial-Knochenschraube ausgebildet ist.
Die Polyaxial-Knochenschraube weist ein Schraubenelement mit einem Gewindeschaft 20 mit einem Knochengewinde und einem kugelsegmentförmigen Kopf 21 auf, der mit einem Aufnahmeteil 22 verbunden ist. Das Aufnahmeteil 22 hat an seinem einen Ende eine axialsymmetrisch ausgerichtete erste Bohrung 23, deren Durchmesser größer als der des Gewindeabschnitts des Gewindeschafts 20 und kleiner als der des Kopfs 21 ist. Ferner weist das Aufnahmeteil 22 eine koaxiale zweite Bohrung 24 auf, die auf dem der ersten Bohrung 23 gegenüberliegenden Ende offen ist und deren Durchmesser so groß ist, daß das Schraubenelement durch das offene Ende mit seinem Gewindeabschnitt durch die erste Bohrung 24 hindurch und mit dem Kopf 21 bis zum Grund der zweiten Bohrung 23 führbar ist. Zwischen der ersten und der zweiten Bohrung ist ein kleiner koaxialer Abschnitt 25 vorgesehen, der unmittelbar an die erste Bohrung 23 angrenzt und zum offenen Ende hin sphärisch ausgebildet ist, wobei der Radius im wesentlichen gleich dem des kugelsegmentförmigen Abschnitts des Kopfs 21 ist.

Das Aufnahmeteil 22 weist ferner eine zur Mittenachse des Teiles symmetrisch angeordnete U-förmige Ausnehmung 26 zum Einlegen des Stabs 100 auf, deren Grund zur ersten Bohrung 23 hin gerichtet ist und durch die zwei freie Schenkel 27, 28 gebildet sind, deren freies Ende 29 den oberen Rand des Aufnahmeteils bildet.

Angrenzend an das freie Ende 29 ist an den Schenkeln 27, 28 ein Innengewinde 30 und ein Außengewinde 31 vorgesehen, die sich bis über eine Strecke bis zu einem vorbestimmten Abstand vom freien Ende 29 erstrecken. An das Innengewinde 30 und das Außengewinde 31 schließt sich ferner an der dem freien Ende 29 abgewandten Seite jeweils ein Freistich 33, 34 an.

Es ist ferner ein zylindrisches Druckelement 35 vorgesehen, dessen Außendurchmesser so gewählt ist, daß das Druckelement vom freien Ende 29 der Schenkel in das Aufnahmeteil einführbar ist und in der Bohrung 24 zu dem Kopf 21 hin verschiebbar ist. An seinem in eingesetzem Zustand dem Kopf 21 zugewandten Ende weist das Druckelement eine sich zu dem Ende hin erweiternde kugelsegmentförmige Ausnehmung 36 auf, deren Kugelradius so gewählt ist, dass das Druckelement in dem in das Aufnahmeteil eingesetzten Zustand den Kopf 21 von oben umfaßt. Am gegenüberliegenden Ende besitzt das Druckelement eine U-förmige Ausnehmung 37, deren Abmessungen so bemessen sind, daß der Stab 100 in in diese einlegbar ist und darin gehalten ist. In dem in das Aufnahmeteil 22 eingesetztem Zustand bildet die U-förmige Ausnehmung 37 des Druckelements 35 einen Kanal in dessen Grund der Stab ruht. In dem gezeigten Ausführungsbeispiel stehen die Seitenwände der U-förmigen Ausnehmung 37 des Druckelements nicht über den eingelegten Stab hervor. Das Druckelement weist ferner eine zentrale Bohrung 38 zum Hindurchführen eines Schraubwerkzeugs zum Einschrauben des Schraubenelements auf.

Zum Fixieren des Kopfes in seiner Winkelstellung und des Stabes ist eine Innenschraube vorgesehen. Ferner ist zur zusätzlichen Fixierung eine Außenmutter vorgesehen.

In montiertem Zustand des Verankerungselements mit eingelegtem Stab und wobei das Druckelement 35 auf den Kopf drückt, weist die Staboberfläche einen Abstand A vom freien Ende 29 des Aufnahmeteils auf. Das Innengewinde 30 und das Außengewinde 31 sind in dem gezeigten Ausführungsbeispiel so bemessen, daß sie sich vom freien Ende 29 her gesehen bis zu einer Position erstrecken, die kleiner ist, als der Abstand A. Der der Grund der U-förmigen Ausnehmung 37 des Druckelements 30 zugewandte untere Rand der Freistiche 33, 34 weist vom freien Ende 29 einen Abstand B aus, der größer ist, als der Abstand A der Staboberfläche vom freien Ende.

Im Betrieb wird in bekannter Weise das Schraubenelement in das Aufnahmeteil eingeführt, dann das Druckelement eingesetzt und dann die Schraube in den Knochen eingeschraubt. Anschließend wird der Stab eingelegt und die Winkelstellung des Aufnahmeteils relativ zum Schraubenlement und der Stab justiert und anschließend durch Einschrauben der Innenschraube und Aufschrauben der Außenmutter in bekannter Weise fixiert.

Die Herstellung des Verankerungslements erfolgt in Bezug auf die Herstellung der Gewinde und der Freistiche des Aufnahmeteils wie bei den ersten beiden Ausführungsformen.

In einer Abwandlung sind wie bei der Abwandlung der zweiten Ausführungsform die Länge von Innengewinde und Außengewinde unterschiedlich und/oder die Freistiche weisen eine unterschiedliche Höhe auf. Alle Abwandlungen der ersten und zweiten Ausführungsform sind auch bei der dritten Ausführungsform möglich.

## Patentansprüche

1. Verankerungselement zur Verwendung in der Wirbelsäulen-oder der Knochenchirurgie mit einem Schaft (1) zur Verankerung in einem Wirbel oder Knochenabschnitt und einem mit dem Schaft verbundenen Aufnahmeteil (2; 22) sowie mit einem einen vorbestimmten Durchmesser (D) aufweisenden Stab (100), wobei das Aufnahmeteil (2; 22) eine U-förmige Ausnehmung (3; 26) mit einem Kanal zur Aufnahme des Stabes (100) und zwei an ihrem Ende freie Schenkel (5, 6; 27, 28) mit einem Gewinde (8, 13; 30, 31) aufweist,
und mit einem Sicherungselement (12, 16), das ein mit dem Gewinde der Schenkel zusammenwirkendes Gewinde aufweist,
wobei bei eingelegtem Stab die den freien Enden (7; 29) zugewandte Staboberfläche einen in axialer Richtung gesehen vorbestimmten Abstand (A) zu den freien Enden (7; 29) besitzt,
**dadurch gekennzeichnet,**
**daß** sich das Gewinde (8, 13; 30, 31) der Schenkel von dem freien Ende aus gesehen gewindeauslauffrei bis zu einem Abstand vom freien Ende aus erstreckt, der kleiner als der oder gleich zu dem vorbestimmten Abstand (A) ist, und das Gewinde dort endet und wobei sich an das Gewinde ein Freistich (9, 14; 33, 34) anschließt, dessen dem Gewinde abgewandter Rand von dem freien Ende einen Abstand (B, B') aufweist, der größer ist, als der vorbestimmte Abstand (A).

2. Verankerungselement nach Anspruch 1, **dadurch gekennzeichnet, daß** die Tiefe des Freistichs (9, 14; 33, 34) der Gewindetiefe entspricht.

3. Verankerungselement nach Anspruch 1 oder 2, **dadurch gekennzeichnet, daß** das Gewinde ein Innengewinde (8; 33) ist und das Sicherungselement als Innenschraube (12) ausgebildet ist.

4. Verankerungselement nach einem der Ansprüche 1 bis 3, **dadurch gekennzeichnet, daß** das Gewinde ein Außengewinde (13; 34) ist und das Sicherungselement als Mutter (16) ausgebildet ist.

5. Verankerungselement nach einem der Ansprüche 1 bis 4, **dadurch gekennzeichnet, daß** das Gewinde (8, 13; 30, 31) als metrisches, als Sägengewinde, als Flachgewinde oder als Gewinde mit einem negativen Lastflankenwinkel ausgebildet ist.

6. Verankerungselement nach einem der Ansprüche 1 bis 5, **dadurch gekennzeichnet, daß** die Verbindung zwischen dem Schaft (1) und dem Aufnahmeteil (2) monoaxial ausgebildet ist.

7. Verankerungselement nach einem der Ansprüche 1 bis 5, **dadurch gekennzeichnet, daß** die Verbindung zwischen dem Schaft (1) und dem Aufnahmeteil (22) polyaxial ausgebildet ist.

8. Verfahren zur Herstellung eines Verankerungselements nach einem der Ansprüche 1 bis 7 mit den Schritten:
- Erzeugen der U-förmigen Ausnehmung (3; 26) mit dem Kanal zur Aufnahme des Stabes vorbestimmten Durchmessers (D), sodass die zwei Schenkel mit freien Enden gebildet werden und bei eingelegtem Stab die den freien Enden zugewandte Staboberfläche den in axialer Richtung gesehen vorbestimmten Abstand (A) zu den freien Enden (7; 29) besitzt,
- Herstellen des Freistichs (9, 14; 33, 34), sodass dessen den freien Enden abgewandter Rand von den freien Enden einen Abstand (B, B') aufweist, der größer als der vorbestimmte Abstand (A) ist, und
- danach Herstellen des Gewindes (8, 13; 30, 31) an den Schenkeln, sodass es sich von den freien Enden aus gesehen gewindeauslauffrei bis zu einem Abstand von den freien Enden erstreckt, der kleiner als der oder gleich zu dem vorbestimmten Abstand (A) ist, und dort endet, wobei sich an das Gewinde der Freistich anschließt, dessen dem Gewinde abgewandter Rand von den freien Enden einen Abstand aufweist, der größer ist, als der vorbestimmte Abstand (A).

## Claims

1. Anchoring element for use in spinal surgery or bone surgery, with a shaft (1) for anchoring in a vertebra or bone section, with a receiving part (2; 22) connected to the shaft, and with a rod (100) that has a predetermined diameter (D), the receiving part (2; 22) having a U-shaped recess (3; 26) with a channel for receiving the rod (100) and two branches (5, 6; 27, 28) which are free at their end and have a thread (8, 13; 30, 31),
and with a securing element (12, 16) that has a thread cooperating with the thread of the branches, so that, when the rod is inserted, the rod surface directed towards the free ends (7; 29) is at a predetermined distance (A) from the free ends (7; 29), seen in the axial direction,
**characterized in that**,
seen from the free end, the thread (8, 13; 30, 31) of the branches extends, without thread runout, from the free end by a distance that is smaller than or equal to the predetermined distance (A), and the thread ends there, the thread being adjoined by an undercut (9, 14; 33, 34) whose edge directed away from the thread is at a distance (B, B'), from the free end, greater than the predetermined distance (A).

2. Anchoring element according to Claim 1,
**characterized in that** the depth of the undercut (9, 14; 33, 34) corresponds to the thread depth.

3. Anchoring element according to Claim 1 or 2,
**characterized in that** the thread is an internal thread (8; 33), and the securing element is designed as an internal screw (12).

4. Anchoring element according to one of Claims 1 to 3, **characterized in that** the thread is an external thread (13; 34), and the securing element is designed as a nut (16).

5. Anchoring element according to one of Claims 1 to 4, **characterized in that** the thread (8, 13; 30, 31) is designed as a metric thread, a buttress thread, a flat thread, or a thread with a negative load-bearing flank angle.

6. Anchoring element according to one of Claims 1 to 5, **characterized in that** the connection between the shaft (1) and the receiving part (2) is monoaxial.

7. Anchoring element according to one of Claims 1 to 5, **characterized in that** the connection between the shaft (1) and the receiving part (22) is multiaxial.

8. Method for producing an anchoring element according to one of Claims 1 to 7, with the following steps:
- generating the U-shaped recess (3; 26) with the channel for receiving the rod of predetermined diameter (D), so that the two branches with free ends are formed and, when the rod is inserted, the rod surface directed towards the free ends is at the predetermined distance (A) from the free ends (7; 29), seen in the axial direction,
- producing the undercut (9, 14; 33, 34) so that its edge directed away from the free ends is at a distance (B, B'), from the free ends, greater than the predetermined distance (A), and
- thereafter producing the thread (8, 13; 30, 31) on the branches so that, seen from the free ends, it extends without thread runout from the free ends by a distance that is smaller than or equal to the predetermined distance (A), and it ends there, the thread being adjoined by the undercut whose edge directed away from the thread is at a distance, from the free ends, greater than the predetermined distance (A).

## Revendications

1. Elément d'ancrage à employer dans la chirurgie de la colonne vertébrale ou des os avec une broche (1) destinée à l'ancrage dans une vertèbre ou une section osseuse et avec une pièce réceptacle (2; 22) raccordée à la broche ainsi qu'avec une tige (100) présentant un diamètre (D) prédéterminé, la pièce réceptacle (2; 22) présentant un creux (3 ; 26) en forme de U avec un canal pour recevoir la tige (100) et deux branches libres (5, 6 ; 27, 28) au niveau de son extrémité avec un filetage (8, 13 ; 30, 31),
et avec un élément d'arrêt (12, 16), qui présente un filetage coopérant avec le filetage des branches, lors de l'insertion de la tige, la surface de tige tournée vers les extrémités libres (7 ; 29) possède une distance (A) prédéterminée, considérée dans la direction axiale, la séparant des extrémités libres (7 ; 29),
**caractérisé en ce que**, le filetage (8, 13 ; 30, 31) des branches, considéré à partir de l'extrémité libre, s'étend sans filet incomplet sur une distance le séparant de l'extrémité libre, distance qui est inférieure ou égale à la distance prédéterminée (A), et le filetage s'y termine, et une entaille dégagée (9, 14 ; 33, 34) se raccordant au filetage, dont le bord faisant dos au filetage présente une distance (B, B') la séparant de l'extrémité libre, qui est supérieure à la distance prédéterminée (A).

2. Elément d'ancrage selon la revendication 1, **caractérisé en ce que**, la profondeur de l'entaille dégagée (9, 14 ; 33, 34) correspond à la profondeur du filetage.

3. Elément d'ancrage selon la revendication 1 ou 2, **caractérisé en ce que** le filetage est un filetage intérieur (8 ; 33) et l'élément d'arrêt est formé en tant que vis intérieure (12).

4. Elément d'ancrage selon l'une quelconque des revendications 1 à 3, **caractérisé en ce que**, le filetage est un filetage externe (13 ; 34) et l'élément d'arrêt est formé en tant qu'écrou (16).

5. Elément d'ancrage selon l'une quelconque des revendications 1 à 4, **caractérisé en ce que** le filetage (8, 13 ; 30, 31) est formé en tant que filetage métrique, filetage en dents de scie, filetage carré ou filetage avec un angle négatif de flanc de charge.

6. Elément d'ancrage selon l'une quelconque des revendications 1 à 5, **caractérisé en ce que** le raccordement entre la broche (1) et la pièce réceptacle (2) est formé de façon mono-axiale.

7. Elément d'ancrage selon l'une quelconque des revendications 1 à 5, **caractérisé en ce que** le raccordement entre la broche (1) et la pièce réceptacle (2) est formé de façon poly-axiale.

8. Procédé de fabrication d'un élément d'ancrage selon l'une quelconque des revendications 1 à 7, avec les étapes consistant :
- à produire le creux (2; 26) en forme de U avec le canal pour recevoir la tige d'un diamètre prédéterminé (D), de sorte que les deux branches sont formées avec des extrémités libres et lorsque la tige est insérée, la surface de tige tournée vers les extrémités libres possède une distance prédéterminée (A) la séparant des extrémités libres (7; 29), considérée dans la direction axiale,
- à fabriquer l'entaille dégagée (9, 14; 33, 34), de sorte que son bord faisant dos aux extrémités libres présente une distance (B, B') le séparant des extrémités libres, qui est supérieure à la distance prédéterminée (A), et
- à fabriquer ensuite le filetage (8, 13; 30,31) sur les branches, de sorte qu'il s'étend sans filet incomplet, considéré à partir des extrémités libres, jusqu'à une distance le séparant des extrémités libres, qui est inférieure ou égale à la distance prédéterminée (A), et s'y termine, en se raccordant au filetage de l'entaille dégagée, dont le bord faisant dos au filetage présente une distance le séparant des extrémités libres, qui est supérieure à la distance prédéterminée (A).
